(19) Europäisches Patentamt
European Patent Office
Office européen des brevets

(11) **EP 2 910 254 B1**

(12) **EUROPEAN PATENT SPECIFICATION**

(45) Date of publication and mention
of the grant of the patent:
**17.05.2017 Bulletin 2017/20**

(21) Application number: **13862500.9**

(22) Date of filing: **06.12.2013**

(51) Int Cl.:
*A61K 47/32* (2006.01)     *A61K 8/81* (2006.01)
*A61K 8/92* (2006.01)     *A61K 9/06* (2006.01)
*A61K 9/08* (2006.01)     *A61K 47/06* (2006.01)
*A61K 47/34* (2017.01)     *A61K 47/44* (2017.01)
*A61Q 19/00* (2006.01)     *A61K 9/00* (2006.01)
*A61K 47/24* (2006.01)

(86) International application number:
**PCT/JP2013/007185**

(87) International publication number:
**WO 2014/091729 (19.06.2014 Gazette 2014/25)**

(54) **BASE AND EXTERNAL PREPARATION FOR SKIN**

BASIS UND EXTERNE ZUBEREITUNG FÜR DIE HAUT

BASE ET PRÉPARATION EXTERNE POUR LA PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.12.2012 JP 2012270153**

(43) Date of publication of application:
**26.08.2015 Bulletin 2015/35**

(73) Proprietor: **Kanae Technos Co., Ltd.
Kagawa 768-0040 (JP)**

(72) Inventors:
• **KANEMITSU, Yuko
Kanonji-shi
Kagawa 768-0040 (JP)**
• **YASUKI, Daisuke
Kanonji-shi
Kagawa 768-0040 (JP)**

• **FUJITA, Yuko
Kanonji-shi
Kagawa 768-0040 (JP)**
• **TSUJIMURA, Yasushi
Kanonji-shi
Kagawa 768-0040 (JP)**

(74) Representative: **Hinkelmann, Klaus
Patentanwaltskanzlei Hinkelmann
Lyonel-Feininger-Strasse 28
80807 München (DE)**

(56) References cited:
**WO-A1-00/56280     WO-A2-2006/101955
JP-A- H0 782 142     JP-A- 2008 535 956
JP-A- 2012 085 954     US-A1- 2003 235 553**

**Description**

TECHNICAL FIELD

The present invention relates to a base used in an external preparation for skin that administers an active ingredient transdermally, and to an external preparation for skin.

BACKGROUND ART

External preparations for skin, which are dosage forms in which active ingredients are dissolved or dispersed in liquid or ointment (creamy) bases, are applied to skin surfaces to administer active ingredients transdermally.

Transdermal administration of active ingredients takes a long time, and thus the external preparations for skin are required to remain attached to the skin surface for a long time.

An external preparation for skin (bath film agent) that, after application to a skin surface, can form a coating film has been recently developed (see, for example, Patent Document 1).

PRIOR ART DOCUMENT

PATENT DOCUMENT

US 2003/0235553 A1 describes a composition comprising at least one liquid fatty phase comprising

(i) at least one oil structured with at least one structuring polymer consisting of a polymer (homopolymer or copolymer) with a weight-average molecular mass ranging from 500 to 500,000, containing at least one moiety comprising:

at least one polyorganosiloxane group, consisting of from 1 to 1000 organosiloxane units in the chain of the moiety or in the form of a graft, and at least two groups capable of establishing hydrogen interactions, chosen from ester, amide, sulphonamide, carbamate, thiocarbamate, urea, thiourea, oxamido, guanamido and biguanamido groups,
and combinations thereof, on condition that at least one of the groups is other than an ester group, the polymer being solid at 25°C and soluble in said oil at a temperature of from 25 to 250°C, and

(ii) at least one film-forming polymer,

said oil having an affinity with said structuring polymer and optionally said film-forming polymer, and the liquid fatty phase, the structuring polymer, and the film-forming polymer forming a physiologically acceptable medium.

WO 2006/101955 A2 and JP 2008/535956 A describe a liquid coating composition comprising: (a) 1-40 weight % of siloxane containing polymer; (b) 18-55 weight % of volatile polydimethylsiloxane; (c) 5-55 weight % of aliphatic hydrocarbon; and (d) 0-15 weight % of adjuvant.

WO 00/56280 describes a composition for application to the skin, comprising:

a) 1-40% of siloxane-containing polymer; b) 60-99% of an Alkan-Based Siloxy Polymer Reaction Solvent; and c) 0-15% of adjuvants.

JP 2012/085954 A describes a coating forming composition for alleviating the pain of the skin that is a liquid composition which contains a polymer having coating moldability and a volatile solvent for dispersing or dissolving this polymer. When the composition is applied to the skin, the volatile solvent volatilizes, and the coating comprising the polymer is formed on the skin. The coating is formed by applying the composition onto the skin. The coating forming composition for alleviating the pain of the skin is a liquid composition containing a hydraulic or photosetting monomer or polymer having coating formability. When the composition is applied to the skin, a curing reaction occurs due to moisture in the air or on the skin or due to light to form the coating on the skin.

JP H0782142 A describes an aerosol type tacky composition that is obtained by blending a base composed of an acrylic polymer, a plasticizer and a propellant with a cyclic silicone oil. The propellant is preferably dimethyl ether or a mixture of dimethyl ether and a liquefied natural gas. An aerosol type external preparation comprises a pharmacodynamically effective component and the aerosol type tacky composition. The pharmacodynamically effective composition is one or more agents selected from the group consisting of a skin irritant, an anti-inflammatory analgesic, a skin-protecting agent, a germicidal disinfectant, an agent for suppurative disease and an antifungal agent.

Patent Document 1: Japanese Patent Laid-open Publication No. 2007-015973

## SUMMARY OF THE INVENTION

### PROBLEMS TO BE SOLVED BY THE INVENTION

The external preparation for skin described in Patent Document 1 is a pharmaceutical preparation formed by dissolving nitrocellulose in a solvent, such as 3-methylbutyl acetate, isobutyl acetate, or acetone, and further adding ethyl alcohol thereto. Since nitrocellulose is difficult to handle because of its explosibility, ensuring safety during production of the pharmaceutical preparation is challenging.

Moreover, solvents such as 3-methylbutyl acetate, isobutyl acetate, and acetone are not always preferred ingredients in external preparations for skin because they cause odors and skin irritation.

The present invention has been made in view of the above-described technical problems. An object of the present invention is to provide: a novel base that, after application to a skin surface, can rapidly form a coating film and moreover mitigates occurrence of odors and skin irritation; and an external preparation for skin.

### SOLUTIONS TO THE PROBLEMS

In order to solve the above-described technical problems, the base of the present invention (hereinafter referred to as an "inventive base") is used in an external preparation for skin that administers an active ingredient transdermally, and is characterized by containing at least an acrylic-based synthetic polymer and a volatile oil, wherein the base is in a liquid or ointment form prior to application to a skin surface, and the volatile oil evaporates after application of the base to form a hydrophobic coating film containing the volatile oil in the range of 0 to 60 wt% with respect to 100 parts by weight of the acrylic-based synthetic polymer, wherein the acrylic-based synthetic polymer is at least one selected from alkyl acrylate copolymer, alkyl acrylate copolymer sodium, alkyl acrylate copolymer ammonium, (alkyl acrylate/diacetone acrylamide) copolymer, and (alkyl acrylate/dimethicone) copolymer, and wherein the volatile oil is at least one selected from short chain paraffins and volatile silicone oils.

In a preferred aspect of the present invention, the inventive base has hydrophobicity with a weight change rate (X) of 5.0% or less in accordance with the following test.

<Test>

The weight (D) of the base is measured after immersing a cotton nonwoven fabric (10 cm × 10 cm, weight 60 g/m$^2$) in the base and drying the fabric in a 60°C thermostatic bath for 24 hours. The cotton nonwoven fabric after immersion and drying is next placed in water, stirred for 1 hour, and then taken out of water. After removal of water on the surface of the fabric, the weight (W) of the fabric is measured and the weight change rate (X) is calculated in accordance with the following equation.

$$\text{Equation: } X = (W - D) \times 100/D.$$

In a preferred aspect of the present invention, the volatile oil is selected from a group consisting of cyclic dimethyl silicone oil and isodecane.

In a preferred aspect of the present invention, the acrylic-based synthetic polymer is at least one selected from alkyl acrylate copolymer, alkyl acrylate copolymer sodium and alkyl acrylate copolymer ammonium, and wherein the volatile oil is at least one selected from cylic dimethyl silicone oil and isodecane. In another preferred aspect of the present invention, the acrylic-based synthetic polymer is (alkyl acrylate/dimethicone) copolymer and wherein the volatile oil is isodecane.

The external preparation for skin of the present invention (hereinafter referred to as an "inventive external preparation") is characterized by containing the inventive base and an active ingredient, wherein the base is in a liquid or ointment form prior to application to a skin surface, and the volatile oil evaporates after application of the base to form a hydrophobic coating film containing the volatile oil in the range of 0 to 60 wt% with respect to 100 parts by weight of the acrylic-based synthetic polymer, wherein the acrylic-based synthetic polymer is at least one selected from alkyl acrylate copolymer, alkyl acrylate copolymer sodium, alkyl acrylate copolymer ammonium, (alkyl acrylate/diacetone acrylamide) copolymer, and (alkyl acrylate/dimethicone) copolymer, and wherein the volatile oil is at least one selected from short chain paraffins and volatile silicone oils.

In a preferred aspect of the present invention, the inventive external preparation is used in combination with application of foundation on a hydrophobic coating film formed by application of the inventive external preparation to a skin surface.

EFFECTS OF THE INVENTION

The present invention enables rapid formation of a coating film after application to a skin surface as well as mitigation of occurrence of odors and skin irritation.

EMBODIMENTS OF THE INVENTION

Embodiments of the present invention will be described below by way of examples, but the present invention is not limited to these examples.

[Examples 1 to 7]

The compositions of inventive bases according to Examples 1 to 7 (before application) are shown in Table 1.

[Table 1]

| Ingredient (wt%) | | Example | | | | | | |
|---|---|---|---|---|---|---|---|---|
| | | 1 | 2 | 3 | 4 | 5 | 6 | 7 |
| Acrylic-based resin | Alkyl acrylate copolymer (C10-30) | 40 | | | | | | |
| | Alkyl acrylate copolymer Na (C10-30) | | 40 | | | | | |
| | Alkyl acrylate copolymer ammonium (C10-30) | | | 40 | | | | |
| | (Alkyl acrylate/diacetone acrylamide) copolymer (C10-30) | | | | 40 | | | |
| | (Alkyl acrylate/dimethicone) copolymer (C10-30) | | | | | 10 | 40 | 60 |
| Volatile oil | Volatile silicone oil (cyclic dimethyl silicone oil) | 60 | 60 | | | | | |
| | Short chain paraffin (light isoparaffin (isodecane)) | | | 60 | 60 | 90 | 60 | 40 |

<Properties of Inventive Bases>

It has been found that the inventive bases according to Examples 1 to 7 all maintain a liquid or ointment form before application to a skin surface, and after application to a skin surface, the volatile oil evaporates to rapidly form a coating film on the skin surface.
The inventive base minimizes occurrence of odors and skin irritation because it contains an acrylic resin as an ingredient for forming a coating film and a volatile oil (short chain paraffin (liquid isoparaffin) or volatile silicone oil) as an ingredient that evaporates upon application to a skin surface.

<Coating Film>

The inventive bases according to Examples 1 to 7 were applied in a certain amount to the surface of a glass plate and dried in a 35°C thermostatic bath. The amount of residue of the volatile oil remaining on the coating film formed on the glass surface was measured by comparing the weights of the inventive base at application and after drying. The results showed that, in any of examples, the coating film was formed in which the volatile oil remained in the range of 0 to 60 parts by weight with respect to 100 parts by weight of the acrylic resin. In particular, the coating films formed from the inventive bases according to Examples 3 to 7 containing light isoparaffin as a volatile oil were confirmed to contain the volatile oil in the range of 0 to 30 parts by weight with respect to 100 parts by weight of the acrylic resin.

<Hydrophobicity of Coating Film>

Next, the coating films formed from the inventive bases according to Examples 1 to 7 were evaluated for their hydrophobicity under the following test conditions.

<Test>

The weight (D) of the inventive bases according to Examples 1 to 7 is measured after immersing a cotton nonwoven fabric (10 cm x 10 cm, weight 60 g/m$^2$) in the bases and drying the fabric in a 60°C thermostatic bath for 24 hours. The cotton nonwoven fabric after immersion and drying is next placed in water, stirred for 1 hour, and then taken out of water. After removal of water on the surface of the fabric, the weight (W) of the fabric is measured and the weight change rate (X) is calculated in accordance with the equation: $X = (W - D) \times 100/D$.

The results of the test showed that the inventive bases according to Examples 1 to 7 all had a weight change rate (X) of 5% or less, and thus exhibited high hydrophobicity. In particular, the inventive bases according to Examples 5 to 7 containing (alkyl acrylate/dimethicone) copolymer as an acrylic resin were confirmed to have very high hydrophobicity with a weight change rate (X) of 2% or less.

For reference, a commercially available hydrophobic base (oleaginous base) was subjected to the same test and found to have a weight change rate (X) of 500% or more.

[Example 8]

An inventive external preparation was obtained by incorporating 5 wt% of sodium tocopheryl phosphate (TPNa) as an active ingredient to the inventive base according to Example 6.

The resulting inventive external preparation was evaluated for the skin permeability of the active ingredient in accordance with the following test.

<Test>

A stratum corneum/epidermis layer of the human skin is cut into a circle with 30 mm in diameter and used for the test. The permeability test is carried out in a non-sealed system using a Franz diffusion cell (effective area 3.14 cm$^2$, receptor volume 2.4 mL) as a diffusion cell and a PBS (phosphate buffered saline, pH 7.4) solution as a receptor solution while the diffusion cell is kept at 32°C. The inventive base (1 mL) is applied to the donor side. After 5 minutes, 1 mL of the PBS solution is added to the donor side to wash the layer lightly, and the solution on the donor side is then discarded. This washing procedure is performed another two times (total three times). Thereafter, 1 mL of a solution is sampled from the receptor after 3 hours, 6 hours, and 12 hours (1 mL of the PBS solution is added to the receptor after 3 hours and 6 hours). TPNa in the sampled solution is measured by HPLC (high performance liquid chromatography).

<HPLC Analysis Conditions>

Column: CAPCELL PAK C18, Type MG 5 $\mu$m 4.6 I.D. $\times$ 250 mm (available from Shiseido Co., Ltd.)
Mobile phase: (20 mM acetic acid + 20 mM sodium acetate methanol)/acetonitrile = 7/3
Flow rate: 1.0 mL/min
Temperature: 40°C
Injection volume: 10 $\mu$l

The results of the above test are shown in Table 2.

[Table 2]

|  | After 3 hours | After 6 hours | After 12 hours |
|---|---|---|---|
| Amount of skin permeation ($\mu$g/cm$^2$) | 0.08 | 0.10 | 0.14 |

As shown in Table 2, the inventive external preparation may provide, after formation of the coating film, continuous transdermal administration of the active ingredient even though the surface of the coating film was washed 3 times. This is probably because the inventive base forms a hydrophobic coating film on the skin surface without the base being removed by repeated washing, which allows continuous transdermal administration of the active ingredient.

It is thus found that the inventive external preparation can, after formation of the coating film, transdermally administer the active ingredient over a long time while the inventive external preparation is not removed by bathing, face washing, or the like.

Since the formed coating film is not sticky, the coating film can undergo application of foundation thereon. The coating film can be thus covered with foundation in use. The formed coating film can be easily removed from the skin surface using cleansing oils or the like.

By the way, the inventive external preparation may optionally contain various additives other than the active ingredient, such as moisturizers, preservatives, antioxidants, and pH adjusters.

The active ingredient in the inventive external preparation is not limited to TPNa, and various active ingredients can be used according to the purpose of the inventive external preparation. Examples of active ingredients include corticosteroids, antiphlogistic analgesics, antihypertensive agents, anesthetics, sedative-hypnotics, tranquilizers, hypotensive agents, antibiotics, antibacterial substances, vitamins, antiepileptics, coronary vasodilators, antihistamines, antifungals, sublimate crystals, mentha oil, eucalyptus oil, lavender oil, boric acid solution, physiological saline solution, bitter water, linseed oil, lime water, liver oil, Rivanol solution, potassium permanganate solution, mentha water, creosote, mustard, anti-inflammatory agents, astringents, cooling agents, vitamin agents, hormonal agents, skin care agents such as antihistamines, sebum inhibitors, anti-acne drugs such as keratolytic drugs, animal or plant extracts, such as aloe extract, ginseng extract, and glycyrrhiza extract, and nutrients such as amino acids.

The present invention may be embodied in a variety of other forms without departing from the spirit or main features of the present invention. The foregoing examples are merely illustrative in every respect and should not to be construed as limiting. The scope of the present invention is defined by the claims and is not restricted by the description herein. All modifications and changes within the range of equivalents of the claims are within the scope of the present invention.

INDUSTRIAL APPLICABILITY

The inventive base is suitably used as a base in an external preparation for skin (inventive external preparation) for transdermal administration of an active ingredient.

**Claims**

1. A base used in an external preparation for skin that administers an active ingredient transdermally, comprising at least an acrylic-based synthetic polymer and a volatile oil, wherein
the base is in a liquid or ointment form prior to application to a skin surface, and
the volatile oil evaporates after application of the base to form a hydrophobic coating film containing the volatile oil in the range of 0 to 60 wt% with respect to 100 parts by weight of the acrylic-based synthetic polymer,
wherein the acrylic-based synthetic polymer is at least one selected from alkyl acrylate copolymer, alkyl acrylate copolymer sodium, alkyl acrylate copolymer ammonium, (alkyl acrylate/diacetone acrylamide) copolymer, and (alkyl acrylate/dimethicone) copolymer, and
wherein the volatile oil is at least one selected from short chain paraffins and volatile silicone oils.

2. The base according to claim 1, wherein
the base has hydrophobicity with a weight change rate (X) of 5.0% or less in accordance with the following test:

   <Test>
   where

   the weight (D) of the base is measured after immersing a cotton nonwoven fabric (10 cm $\times$ 10 cm, weight 60 g/m$^2$) in the base and drying the fabric in a 60°C thermostatic bath for 24 hours; and
   the cotton nonwoven fabric after immersion and drying is next placed in water, stirred for 1 hour, and then taken out of water, and after removal of water on the surface of the fabric, the weight (W) of the fabric is measured and the weight change rate (X) is calculated in accordance with the following equation:

$$\text{Equation: } X = (W - D) \times 100/D.$$

3. The base according to claim 1 or 2, wherein
the volatile oil is selected from a group consisting of cyclic dimethyl silicone oil and isodecane.

4. The base according to any one of claims 1 to 3, wherein
the acrylic-based synthetic polymer is at least one selected from alkyl acrylate copolymer, alkyl acrylate copolymer sodium and alkyl acrylate copolymer ammonium, and
wherein the volatile oil is at least one selected from cylic dimethyl silicone oil and isodecane.

**5.** The base according to any one of claims 1 to 3, wherein
the acrylic-based synthetic polymer is (alkyl acrylate/dimethicone) copolymer and wherein the volatile oil is isodecane.

**6.** An external preparation for skin, comprising:

the base according to any one of claims 1 to 5; and
an active ingredient, wherein
the base is in a liquid or ointment form prior to application to a skin surface, and
the volatile oil evaporates after application of the base to form a hydrophobic coating film.

**7.** The external preparation for skin according to claim 6, for use in combination with application of foundation on a hydrophobic coating film formed by application of the external preparation to a skin surface.

**Patentansprüche**

**1.** Eine Basis, die in einer externen Zubereitung für die Haut verwendet wird, welche einen aktiven Inhaltsstoff perkutan verabreicht, umfassend zumindest ein synthetisches Polymer auf Acrylbasis und ein flüchtiges Öl, wobei
die Basis vor der Applikation auf eine Hautoberfläche in einer flüssigen Form oder in Salbenform vorliegt, und
das flüchtige Öl nach der Applikation der Basis verdampft, um einen hydrophoben Beschichtungsfilm zu bilden, der das flüchtige Öl in dem Bereich von 0 bis 60 Gew.-% bezogen auf 100 Gewichtsanteile des synthetischen Polymeren auf Acrylbasis enthält,
wobei das synthetische Polymer auf Acrylbasis aus zumindest einem Polymer der Gruppe Alkylacrylat-Copolymer, Natrium-Alkylacrylat-Copolymer, Ammonium-Alkylacrylat-Copolymer, Alkylacrylat/Diacetonacrylamid-Copolymer und Alkylacrylat/Dimethicon-Copolymer ausgewählt ist, und
wobei das flüchtige Öl zumindest aus kurzkettigen Paraffinen und flüchtigen Silikonölen ausgewählt ist.

**2.** Die Basis nach Anspruch 1, wobei
die Basis eine Hydrophobizität mit einer Gewichtsänderungsrate (X) von 5.0% oder weniger gemäß dem folgenden Test aufweist:

<Test>
wobei

das Gewicht (D) der Basis nach dem Eintauchen eines ungewebten Baumwollgewebes (10 cm x 10 cm, Gewicht 60 g/cm$^2$) in die Basis und Trocknen des Gewebes in einem thermostatischen Bad bei 60°C für 24 Stunden gemessen wird; und
das ungewebte Baumwollgewebe nach dem Eintauchen und Trocknen als nächstes in Wasser eingebracht wird, für 1 Stunde gerührt, und dann aus dem Wasser genommen wird, und nach dem Entfernen des Wassers auf der Oberfläche des Gewebes das Gewicht (W) des Gewebes gemessen wird und die Gewichtsänderungsrate (X) gemäß der folgenden Gleichung berechnet wird:

$$\text{Gleichung: } X = (W-D) \times 100/D.$$

**3.** Die Basis nach Anspruch 1 oder 2, wobei
das flüchtige Öl ausgewählt ist aus einer Gruppe bestehend aus cyclischem Dimethylsilikonöl und Isodekan.

**4.** Die Basis nach einem der Ansprüche 1 bis 3, wobei
das synthetische Polymer auf Acrylbasis aus zumindest einem aus der Gruppe von Alkylacrylat-Copolmyer, Natrium-Alkylacrylat-Copolymer und Ammonium-Alkylacrylat-Copolymer ausgewählt ist, und
wobei das flüchtige Öl aus zumindest einem von cyclischem Silikonöl und Isodekan ausgewählt ist.

**5.** Die Basis nach einem der Ansprüche 1 bis 3, wobei
das synthetische Polymer auf Acrylbasis ein Alkylacrylat/Dimethicon-Copolymer ist und wobei das flüchtige Öl Isodekan ist.

**6.** Eine externe Zubereitung für die Haut, umfassend:

die Basis nach einem der Ansprüche 1 bis 5; und
einen aktiven Inhaltsstoff, wobei
die Basis vor der Applikation auf eine Hautoberfläche in einer flüssigen Form oder in Salbenform vorliegt, und
das flüchtige Öl nach der Applikation der Basis verdampft, um einen hydrophoben Beschichtungsfilm zu bilden.

**7.** Die externe Zubereitung für die Haut nach Anspruch 6, zur Verwendung in Kombination mit einer Applikation einer Fundierung auf einen hydrophoben Beschichtungsfilm, der durch Applikation der externen Zubereitung auf eine Hautoberfläche gebildet ist.

**Revendications**

**1.** Base qui est utilisé dans une préparation externe pour la peau administrant par voie transdermique un ingrédient actif, comprenant au moins un polymère synthétique à base d'acrylique et une huile volatile, dans laquelle
la base existe d'une forme liquide ou pommade avant l'application sur une surface de peau, et
l'huile volatile s'évapore après l'application de la base pour former un film de revêtement hydrophobe contenant l'huile volatile dans la plage de 0 à 60% en poids par rapport à 100 parties en poids du polymère synthétique à base d'acrylique,
dans laquelle le polymère synthétique à base d'acrylique est au moins un polymère choisi parmi un copolymère d'acrylate d'alkyle, un copolymère d'acrylate d'alkyle à base de sodium, un copolymère d'acrylate d'alkyle à base d'ammonium, un copolymère d'acrylate d'alkyle/diacétone d'acryleamide, et un copolymère d'acrylate d'alkyle/diméthicone, et
dans laquelle l'huile volatile est au moins une choisi parmi des paraffines à chaîne courte et des huiles de silicone volatiles.

**2.** La base selon la revendication 1, dans laquelle
la base a une hydrophobicité avec un taux de changement de poids (X) de 5,0% ou moins selon le test suivant:

<Test>
dans lequel

le poids (D) de la base est mesuré après l'immersion d'un tissu de coton non-tissé (10 cm x 10 cm, poids 60 g/cm$^2$) dans la base et le séchage du tissu dans un bain thermostatique à 60°C pendant 24 heures; et le tissu de coton non-tissé après l'immersion et le séchage est ensuite introduit dans l'eau, agité pendant une heure, et ensuite retiré de l'eau, et après élimination de l'eau à la surface du tissu, le poids (W) du tissu est mesuré et le taux de variation de poids (X) est calculé selon l'équation suivante:

$$\text{Equation: } X = (W-D) \times 100/D.$$

**3.** La base selon la revendication 1 ou 2, dans laquelle
l'huile volatile est choisie parmi d'un groupe constitué de diméthylsilicone cyclique et isodécane.

**4.** La base selon l'une quelconque des revendications 1 à 3, dans laquelle
le polymère synthétique à base d'acrylique est un polymère choisi parmi un copolymère d'acrylate d'alkyle, un copolymère d'acrylate d'alkyle à base de sodium et un copolymère d'acrylate d'alkyle à base d'ammonium, et
dans laquelle l'huile volatile est au moins une choisi parmi d'huile silicone cyclique et d'isodécane.

**5.** La base selon l'une quelconque des revendications 1 à 3, dans laquelle
le polymère synthétique à base d'acrylique est un copolymère d'acrylate d'alkyle/diméthicone et dans laquelle l'huile volatile est isodécane.

**6.** Une préparation externe pour la peau, comprenant:

la base selon l'une quelconque des revendications 1 à 5; et

un ingrédient actif, dans laquelle
la base existe dans une forme liquide ou de pommade avant l'application à une surface de peau, et
l'huile volatile s'évapore après l'application de la base pour former un film de revêtement hydrophobe.

7. La préparation externe pour la peau selon la revendication 6, pour une utilisation en combinaison avec une application d'une fondation sur un film de revêtement hydrophobe formé par application de la péparation externe sur une surface de la peau.

**EP 2 910 254 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030235553 A1
- WO 2006101955 A2
- JP 2008535956 A
- WO 0056280 A
- JP 2012085954 A
- JP H0782142 A
- JP 2007015973 A